# EUROPÄISCHE PATENTSCHRIFT

(11) **EP 2 292 187 B1**
(45) Veröffentlichungstag und Bekanntmachung des Hinweises auf die Patenterteilung: **23.05.2012**
(21) Anmeldenummer: 10002117.9
(22) Anmeldetag: 02.03.2010
(51) Int. Cl.: A61F 2/36, A61F 2/48

(54) **Wachstumsprothese**
Growth prosthesis
Prothèse de croissance

(30) Priorität: 04.03.2009 DE 102009011661
(43) Veröffentlichungstag der Anmeldung: 09.03.2011
(73) Patentinhaber: Wittenstein AG, 97999 Igersheim (DE)
(72) Erfinder: Stauch, Roman, 97959 Assamstadt (DE)
(74) Vertreter: Arat, Dogan

(56) Entgegenhaltungen:
- EP-A2- 1 611 868
- US-A- 5 626 579

## Beschreibung

Die Erfindung betrifft eine Wachstumsprothese umfassend eine Knochenprothese, insbesondere Gelenkersatzteil, einen Prothesenschaft, ein Schaftverankerungselement und ein zur Verlängerung der Wachstumsprothese vorgesehenes Element mit entsprechendem Antrieb, gemäß Oberbegriff des Patentanspruchs 1.

Aus dem Stand der Technik sind eine Reihe von Wachstumsprothesen bekannt.

In diesem Zusammenhang wird zunächst auf die EP 1 611 868 A2 hingewiesen, welche eine anpassbare orthopädische Prothese offenbart. Weiter wird auf die US 5,626,579 hingewiesen, welche eine Knochenversatz- und Verlängerungsvorrichtung offenbart.

Wachstumsprothesen bestehen meist aus einem verlängerbaren Prothesenteil und einem Schaftverankerungselement, welches im Knochen fixiert wird. Ein elektrischer Antrieb befindet sich bei derartigen Vorrichtungen im Prothesenteil. Als elektrischer Antrieb kommen meist Spindelrollensysteme zum Einsatz. In der EP 0 776 432 BI wird eine derartige Antriebseinheit offenbart. Die Vorrichtung wandelt dabei mit von einer Antriebswelle aus einem ortsfesten Antriebsgehäuse heraus angetriebenen umfangsmäßig auf gleichem Abstand gehaltenen Planetenrollen und einem durch die Planetenrollen axial verschiebbaren Schubkörper, eine Dreh- in eine Axialbewegung um.

Aufgrund der Bewegung der Antriebseinheit wird das teleskopierbare Element der Prothese herausbewegt und die Prothese verlängert. Dabei kann das Schaftverankerungselement verschiedene Längen und Querschnitte aufweisen und modular aufgebaut sein, damit es den unterschiedlichen Markraumdimensionen angepasst werden kann.

Aus der EP 1 371 346 B1 ist beispielsweise eine implantierbare Prothese zum Ersatz des menschlichen Hüft- oder Kniegelenks und der benachbarten Knochenschaftanteile bekannt, wobei die Prothese ein Gelenkteil, ein Schaftverankerungsteil und einen zwischen dem Gelenkteil und dem Schaftverankerungsteil angeordneten Schaftersatzteil aufweist. Dabei verfügen das Schaftersatzteil und das künstliche Gelenk jeweils eine durchgehende zentrale Bohrung mit fluchtender Ausrichtung zueinander. Das Schaftverankerungsteil wird bei der vorliegenden Erfindung von einem Distraktionsmarknagel gebildet und wird in die zentralen Bohrungen eingesetzt. Nach der gewünschten Verlängerung des Knochens mittels Kallusdistraktion, also der Bildung neuen Knochengewebes, müssen derartige Marknägel meist wieder entfernt werden und durch einen Stabilisator ersetzt werden, da die Marknägel nicht genügend Stabilität für einen dauerhaften Verbleib der Prothese aufweisen.

In der DE 199 06 423 A1 wird des Weiteren ein aktiver Marknagel zur Distraktion von Knochenteilen offenbart, welcher aus zwei gegeneinander bewegbaren Elementen, Prothesen oder dergleichen mit mindestens einem elektrisch betriebenen Antriebselement besteht. Die für das Antriebselement nötige elektrische Energie, wird dem Marknagel über zumindest ein wiederlösbares Steckerelement zugeführt. Sollte der Marknagel in die Prothese integriert sein, ist es bei dieser Erfindung besonders von Vorteil, dass nach Abschluss der gewünschten Distraktion nur der elektrische Steckverbinder entfernt werden muss und nicht der gesamte Marknagel. Allerdings besteht bei dieser Vorrichtung weiterhin das Problem, dass zur Einbringung der Prothese aufgrund der vorgegebenen Baulänge ein nicht unerheblich großer Anteil gesunden Knochens entfernt werden muss.

Nachteilig am aufgezeigten Stand der Technik ist zum einen der relativ große Durchmesser von Wachstumsprothesen mit Planetrollenantrieben. Derartige Prothesenmodelle sind nur im eingeschränkten Maße bei der medizinischen Versorgung zu ersetzender Hüft- und Kniegelenke bei Kindern und Jugendlichen geeignet. Auch die lange Mindestprothesenlänge, welche durch die relativ große Länge des Antriebes vorgegeben wird, stellt einen erheblichen Nachteil dar, da bei Kinder mit kleineren Knochen aufgrund der langen und vorgegebenen Prothesenlänge mehr Knochen als nötigt entfernt werden muss, um den Einbauraum für die zu implantierende Prothese zu schaffen.

Aus dem Vorgenannten ist es daher die Aufgabe der vorliegenden Erfindung, eine Wachstumsprothese zu schaffen, die vom Prinzip der Kallusdistraktion abweicht und welche gleichzeitig eine reduzierte Prothesenlänge sowie einen reduzierten Prothesendurchmesser aufweist, um eine einfachere Anwendung derartiger Prothesen bei Kindern und Jugendlichen oder kleinwüchsigen Personen zu schaffen.

Die Lösung der Aufgabe der Erfindung erfolgt durch eine Wachstumsprothese umfassend einen Prothesenschaft, ein Gelenkersatzteil, ein Schaftverankerungselement und ein zur Verlängerung der Wachstumsprothese vorgesehenes Element mit entsprechendem Antrieb, gemäß Oberbegriff des Patentanspruchs 1, wobei die Unteransprüche mindestens zweckmäßige Ausgestaltungen und Weiterbildungen darstellen.

Das als Gelenkersatzteil bezeichnete Element der Wachstumsprothese weist eine derartige Form und Geometrie auf, dass es einem zu ersetzenden Gelenk, z.B. Knie, Hüfte, etc. gleicht, also ein Gelenkersatzteil darstellt. Typischerweise ist dieses Element aus Implantatstahl oder ähnlich bekannten Materialen gefertigt.

Das Gelenkersatzteil ist dabei im Prothesenschaft eingesetzt, wobei diese gegeneinander verdrehgesichert sind.

Das Schaftverankerungselement stellt ein zum Gelenkersatzteil und Prothesenschaft gegenüberliegendes Element der Wachstumsprothese dar und wird im Markraum des Knochens eingeführt und fixiert. Nach der Entfernung eines Hüft- oder Kniegelenks aufgrund eines Knochentumors, wird möglicherweise zunächst keine Verkürzung des Beines festgestellt, wodurch eine Distraktion des Knochens zunächst nicht notwendig ist. Bei Kindern und Jugendlichen stellen sich jedoch aufgrund des natürlichen Knochenwachstums bereits nach kürzester Zeit Beinlängendifferenzen ein, die folglich medizinisch behandelt werden müssen.

Deshalb weist die Wachstumsprothese ein Element mit Antrieb auf, welches zur Verlängerung der gesamten Prothese dient und postoperative Längendifferenzen der Gliedmaße ausgleichen kann.

Erfindungsgemäß handelt es sich bei dem zur Verlängerung der Wachstumsprothese vorgesehenen Element um ein Spindelelement, welches im Schaftverankerungselement angeordnet ist.

Aufgrund dieser Anordnung verringert sich die Länge sowie der Durchmesser der Wachstumsprothese um einen erheblichen Betrag und ist deshalb bei der Behandlung von Kindern und Jugendlichen mit kleineren Knochen besonders geeignet.

Die Wachstumsprothese ist ferner modular aufgebaut. D. h. die einzelnen Bestandteile der Prothese können gemäß den anatomischen Gegebenheiten ausgewählt und kombiniert werden. So können die einzelnen Prothesenelemente gegeneinander ausgetauscht werden und entsprechend des Durchmessers und der Länge der vorliegenden Knochen eingesetzt werden.

Aufgrund des modularen Aufbaus der Wachstumsprothese ist es außerdem möglich, das Spindelelement und den Antrieb nach Abschluss des Verlängerungsprozesses aus der Prothese einfach zu entfernen.

Es ist auch möglich, die nach der Tumorentfernung eingesetzte Prothese zunächst ohne Antrieb zu implantieren und diesen erst nach erfolgreichem Ende der Chemotherapie einzusetzen.

Um das Spindelelement im Schaftverankerungselement einsetzen zu können, verfügt das Schaftverankerungselement über eine Bohrung und zwar entsprechend der Länge und Art des Spindelelements, wobei sich die Bohrung nahezu vollständig über die Länge des Schaftverankerungselements erstreckt.

Ein Innengewinde zur Aufnahme und Abstützung des Spindelelements kann direkt im Schaftverankerungselement, in einer Schraube zur Verbindung von Schaftverankerungselement und Prothese oder in einem formschlüssig eingesetzten Gewindeelement angebracht sein.

Der Durchmesser der Bohrung des Schaftverankerungselements ist hierbei so gewählt, dass ein bezüglich der Kraftaufbringung zur Verlängerung der Wachstumsprothese ausreichend großes Spindelelement aufgenommen werden kann, jedoch keine wesentliche Verringerung der Festigkeit des Schaftverankerungselements eintritt.

Das Außengewinde des Spindelelements entspricht bei der erfindungsgemäßen Vorrichtung hinsichtlich der Gewindesteigung und der Gewindeabmessung dem des im Schaftverankerungselement angebrachten Gewindes. Es ist derart ausgestaltet, dass eine gewünschte Kontinuität der Bewegung, die notwendige Spielfreiheit und ein sicheres Beibehalten eines einmal erreichten Verstellwegs gewährleistet werden.

Der Antrieb des Spindelelements ist zweckmäßigerweise im Gelenkersatzteil platziert. Die platzsparende Anordnung des Antriebes kann durch eine entsprechend große Bohrung am Ende des Gelenkersatzteils realisiert werden, ohne eine wesentliche Verringerung der Stabilität in Kauf nehmen zu müssen, zumal es sich bei dem Material der Knochenprothese um, wie bereits erwähnt, Implantatstahl, Keramik und ähnlichen Materialien handelt.

Vorzugsweise umfasst der Antrieb des Spindelelements einen Motor mit Untersetzungsgetriebe.

Denkbar sind in diesem Zusammenhang Ausführungsformen des Antriebes als piezoelektrischer Aktuator, Formgedächtnisaktuator, als elektromagnetischer oder magnetischer Antrieb.

In einer bevorzugten Ausführungsform der Erfindung ist in die Bohrung am Ende des Schaftverankerungselments formschlüssig ein Spindel-Gegenstück in Form eines Gewindeelements mit einer Durchgangsbohrung mit Innengewinde angebracht.

Das Gewindeelement dient hierbei als Stützmittel des Spindelelements.

Das Gewindeelement kann dabei beispielsweise aus Implantatstahl, Keramik oder Kunststoff gefertigt sein. Diese Materialen gewährleisten die nötige Stabilität und Festigkeit des Gewindeelements.

Vorzugsweise weist das Gewindeelement eine gegen Verdrehen sichernde Außenkontur auf. Somit bildet das Gewindeelement eine formschlüssige Steckverbindung, welche eine einfache Aus- und Einbringung des Spindelelements gewährleistet, indem vor dem Einsetzen des Spindelelements das Gewindeelement auf diese aufgebracht wird und anschließend ohne umständliches Hineindrehen im Schaftverankerungsteil per Formschluss arretiert werden kann. Aufgrund der Steckverbindung und dem modularen Aufbau der Wachstumsprothese kann der Antrieb mit Spindelelement nach vollständiger Verlängerung der Prothese separat entfernt werden.

Der Einbau des Antriebs in die Wachstumsprothese kann folglich auch erst einige Zeit nach der eigentlichen Implantation der Wachstumsprothese erfolgen, sofern die medizinische Indikation zu einer Verlängerung erst zu einem späteren Zeitpunkt festgestellt wird.

Die Wachstumsprothese ist vor allem bei der medizinischen Versorgung von Knochentumoren bei Kleinkindern und jugendlichen Patienten geeignet, deren Wachstumsphase noch nicht abgeschlossen ist und über kleinere Knochen verfügen und daher speziell auf ihre Bedürfnisse angepasste Prothesen benötigen. Das Auftreten von Knochentumoren führt in vielen Fällen zu einer Amputation des nahe gelegenen Hüft-, Knie-, Ellenbogen-, Hand- oder Schultergelenks (etc.). Durch das Gelenkersatzteil wird zum einen das fehlende Gelenk ersetzt und aufgrund der verlängerbaren Ausführungsform in Form eines Spindelelements eine Anpassung der Längendifferenz der Gliedmaße in Folge des natürlichen und noch nicht abgeschlossenen Knochenwachstums gewährleistet. Aufgrund der schmalen und kurzen Ausgestaltung der Prothese muss bei der Implantation der Wachstumsprothese weniger gesundes Knochenmaterial entfernt werden.

Ein weiterer Vorteil durch die geringe Resektionslänge ist das günstige Verhältnis von Knochen zu Prothese, was eine höhere Stabilität zufolge hat.

Es ist jedoch auch ein Einsatz der Wachstumsprothese bei erwachsenen Patienten denkbar, welche aufgrund von angeborenen, krankheitsbedingten oder unfallbedingten Beinlängendifferenzen behandelt werden. Die kompakte und verkleinerte Ausführungsform der Prothese kann auch in derartigen Anwendungsbereichen leichter implantiert und Entfernungen des Antriebs oder des Spindelelements einfacher ausgeführt werden.

Die Erfindung soll nachstehend anhand eines Ausführungsbeispiels sowie unter Zuhilfenahme von Figuren näher erläutert werden.

Hierbei zeigen:
- Fig. 1: eine Prinzipdarstellung der erfindungsgemäßen Wachstumspro- these;
- Fig. 2: eine Detaildarstellung der Schraube mit Gewindeelement und
- Fig. 3: eine Draufsicht der Schraube mit Gewindeelement.

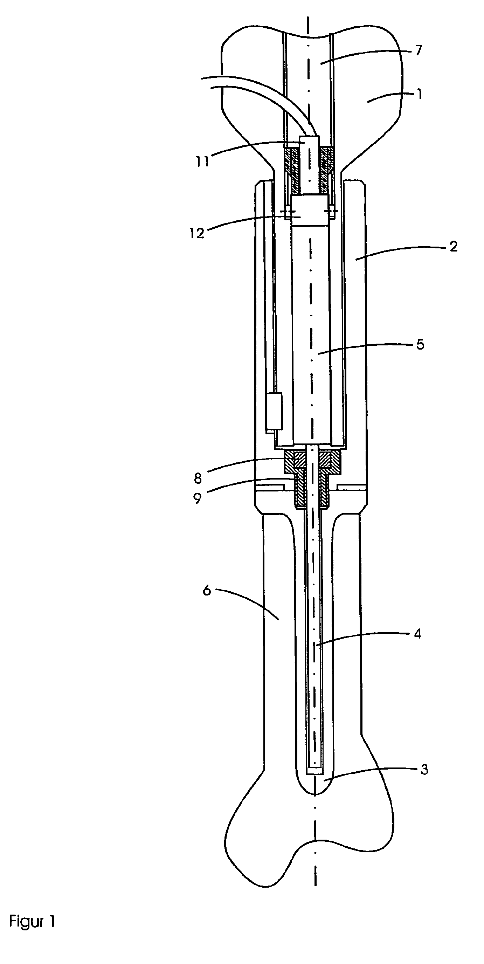

Wie in Fig. 1 verdeutlicht wird, besteht die Wachstumsprothese aus dem Gelenkersatzteil 1, dem Prothesenschaft 2, dem Schaftverankerungselement 3, dem Spindelelement 4 und dem zum Spindelelement 4 zugehörigen Antrieb 5.

Das Gelenkersatzteil 1 ist dabei im nach oben offenen Prothesenschaft 2 angeordnet.

Das Gelenkersatzteil 1 ersetzt mindestens Teile des entfernten Knie- oder Hüftgelenks und ist aus Implantatstahl, Keramik oder ähnlichen Materialien gefertigt und nimmt dabei in einer vorgesehenen Bohrung 7 den Antrieb 5 des Spindelelements 4 auf. Die Größe der Bohrung 7 und des Antriebs 5 sind dabei derart gewählt, dass es zu keiner wesentlichen Verringerung der Stabilität der Prothese führt.

Das Spindelelement 4 befindet sich im eingefahrenen Zustand in einer im Schaftverankerungselement 3 angebrachten Bohrung. Das Spindelelement 4 füllt dabei einen sehr großen Teil der Länge des Schaftverankerungselements 3, welches im Knochen 6 des Patienten fixiert ist, aus. Aufgrund dieser Bauweise kommt es zu einer erheblichen Verkürzung der Gesamtlänge der Wachstumsprothese sowie des Durchmessers im Bereich des Antriebs.

Das Außengewinde des Spindelelements 4 entspricht bezüglich der Gewindesteigung und der Gewindeabmessung dem Gewinde 10 des Gewindeelements 8 und dient der Abstützung des Spindelelements 4, der Umsetzung der rotativen in eine lineare Bewegung und gewährleistet die notwendige Spielfreiheit und das sichere Beibehalten eines einmal erreichten Verstellwegs durch entsprechende Spindelsteigung.

Gelenkersatzteil 1 und Prothesenschaft 2 sind gegeneinander verdrehgesichert. Der Antrieb 5 muss axial sowie verdrehsicher im Gelenkersatzteil gelagert werden.

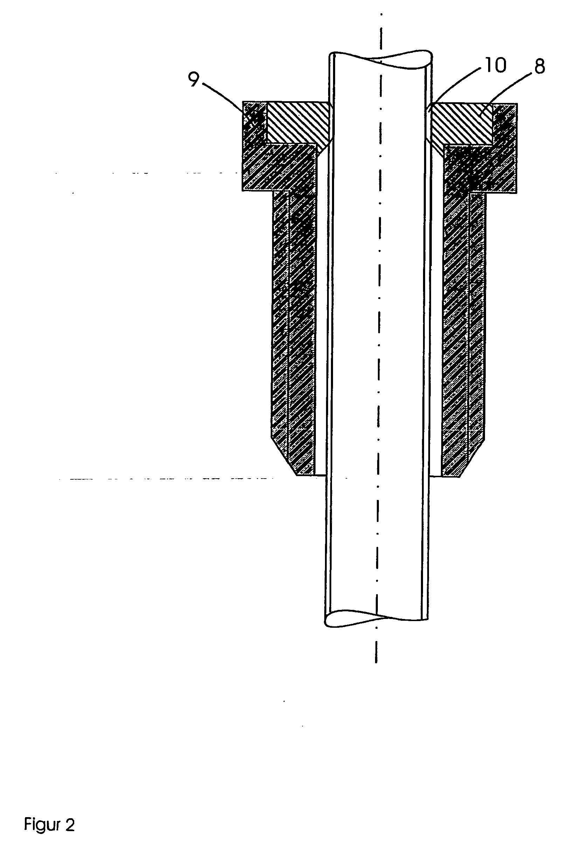

In einer Bohrung am Ende des Schaftverankerungselements 3 bzw. des Prothesenschafts 2 ist eine Schraube 9 mit Gewindeelement 8 eingebracht, welches als ein zum Spindelelement 4 dienendes Gegenstück dient. (Fig. 2).

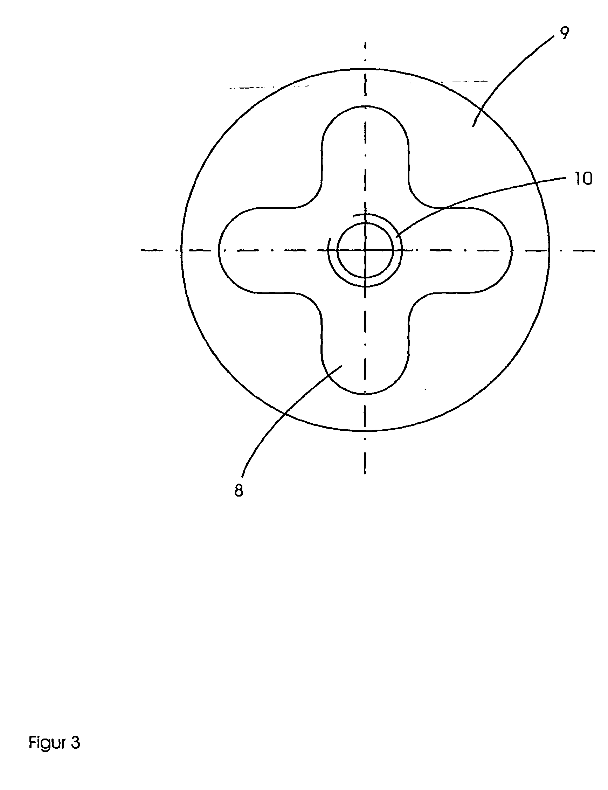

Wie in Fig. 3 dargestellt, weist die Schraube 9 eine kleeblattförmige Ausnehmung oder Vertiefung auf, in welche das Gewindeelement 8 mit Gewinde 10 formschlüssig eingesetzt ist und zur Festlegung des Spindelelements 4 gegenüber dem Schaftverankerungselement 3 dient.

Durch diese formschlüssige Steckverbindung kann eine einfache Aus- und Einbringung der Spindel gewährleistet werden, indem vor dem Einsetzen des Spindelelements 4 das Gewindeelement 8 auf diese aufgedreht wird und anschließend ohne umständliches Hineindrehen des Spindelelements 4 im Schaftverankerungsteil 3 per Formschluss arretiert werden kann. Die Ausbringung erfolgt in gleicher Weise.

So kann das Spindelelement 4 sowie der Antrieb 5, welcher im dargestellten Beispiel in Form eines Motors 11 mit einem Getriebe 12 ausgeführt ist, einfach durch die Bohrung 7 des Gelenkersatzteils 1 entfernt werden, sofern dieser für eine weitere Behandlung nicht mehr benötigt wird. Die erreichte Distraktionsstrecke kann durch einsetzen eines Dummies/Platzhalters aufrechterhalten werden.

### Bezugszeichenliste

- 1: Gelenkersatzteil
- 2: Prothesenschaft
- 3: Schaftverankerungselement
- 4: Spindelelement
- 5: Antrieb
- 6: Knochen
- 7: Bohrung im Gelenkersatzteil
- 8: Gewindeelement
- 9: Schraube mit kleblattförmiger Vertiefung
- 10: Gewinde
- 11: Motor
- 12: Getriebe

## Patentansprüche

1. Wachstumsprothese umfassend ein Gelenkersatzteil (1), einen Prothesenschaft (2), ein Schaftverankerungselement (3) und ein zur Verlängerung der Wachstumsprothese vorgesehenes Element mit entsprechendem Antrieb (5), wobei zur Verlängerung ein Spindelelement (4) vorgesehen ist, welches im Schaftverankerungselement (3) angeordnet ist,
**dadurch gekennzeichnet, dass**
der Antrieb (5) des Spindelelements (4) im Gelenkersatzteil (1) integriert ist, wobei in eine Bohrung im Schaftverankerungselement (3) ein als zum Spindelelement (4) dienendes Gegenstück in Form einer Schraube (9) mit Gewindeelement (8) eingebracht ist, wobei das Gewindeelement (8) formschlüssig mit der Schraube (9) verbunden ist und zur Festlegung des Spindelelementes (4) gegenüber dem Schaftverankerungselement (3) dient.

2. Wachstumsprothese nach Anspruch 1, **dadurch gekennzeichnet, dass** die Wachstumsprothese modular aufgebaut ist.

3. Wachstumsprothese nach Anspruch 1, 2 oder 3, **dadurch gekennzeichnet, dass** das Spindelelement (4) und/oder der Antrieb (5) nach erfolgter Implantation einsetzbar und wiederentfernbar ist.

4. Wachstumsprothese nach einem der vorangegangenen Ansprüche, **dadurch gekennzeichnet, dass** das Schaftverankerungselement (3) eine Bohrung zur Aufnahme des Spindelelements (4) aufweist.

5. Wachstumsprothese nach Anspruch 4, **dadurch gekennzeichnet, dass** der Durchmesser der Bohrung des Schaftverankerungselements (3) so groß gewählt ist, dass ein bezüglich der Kraftaufbringung zur Verlängerung der Wachstumsprothese ausreichend großes Spindelelement (4) aufgenommen werden kann, ohne dass eine wesentliche Verringerung der Festigkeit des Schaftverankerungselements (3) eintritt.

6. Wachstumsprothese nach einem der vorigen Ansprüche, **dadurch gekennzeichnet, dass** der Antrieb (5) des Spindelelements (4) aus einem Motor (11) mit Getriebe (12) aufgebaut ist.

7. Wachstumsprothese nach einem der vorigen Ansprüche, **dadurch gekennzeichnet, dass** der Antrieb (5) des Spindelelements (4) durch einen piezoelektrischen Motor gebildet wird.

8. Wachstumsprothese nach einem der vorigen Ansprüche, **dadurch gekennzeichnet, dass** der Antrieb (5) des Spindelelements (4) einen Formgedächtnisaktuator aufweist.

9. Wachstumsprothese nach einem der vorigen Ansprüche, **dadurch gekennzeichnet, dass** das Spindelement (4) im Gewindeelement (8) der Schraube (9) verläuft.

10. Wachstumsprothese nach einem der vorigen Ansprüche, **dadurch gekennzeichnet, dass** das Gewindeelement (8) mit Gewinde (10) als Stützmittel des Spindelelements (4) dient.

11. Wachstumsprothese nach einem der vorigen Ansprüche, **dadurch gekennzeichnet, dass** das Gewindeelement (8) aus Implantatstahl oder Keramik oder Kunststoff gefertigt ist.

12. Wachstumsprothese nach einem der vorangegangenen Ansprüche, **dadurch gekennzeichnet, dass** der Antrieb (4) des Spindelelements bei weiterhin andauernder Implantation wahlweise entfernbar oder einbaubar ist.

## Claims

1. A growing prosthesis comprising a joint replacement part (1), a prosthesis stem (2), a stem-anchoring element (3) and an element provided for lengthening the growing prosthesis with a corresponding drive (5), with a spindle element (4) being provided for lengthening, which element is arranged in the stem-anchoring element (3),
**characterised in that**
the drive (5) of the spindle element (4) is integrated in the joint replacement part (1), with a mating piece, in the form of a screw (9) with threaded element (8), serving as a the spindle element (4) being introduced into a bore in the stem-anchoring element (3), the threaded element (8) being connected in positive manner to the screw (9), and serving for fixing the spindle element (4) relative to the stem-anchoring element (3).

2. A growing prosthesis according to Claim 1, **characterised in that** the growing prosthesis is of modular construction.

3. A growing prosthesis according to Claim 1 or 2, **characterised in that** the spindle element (4) and/or the drive (5) can be inserted and removed again once implantation has taken place.

4. A growing prosthesis according to one of the preceding claims, **characterised in that** the stem-anchoring element (3) has a bore for receiving the spindle element (4).

5. A growing prosthesis according to Claim 4, **characterised in that** the diameter of the bore of the stem-anchoring element (3) is selected to be so large that a spindle element (4) which is sufficiently large with respect to the application of force for lengthening the growing prosthesis can be received without a significant reduction in the strength of the stem-anchoring element (3) occurring.

6. A growing prosthesis according to one of the above claims, **characterised in that** the drive (5) of the spindle element (4) is constructed from a motor (11) with gear mechanism (12).

7. A growing prosthesis according to one of the above claims, **characterised in that** the drive (5) of the spindle element (4) is formed by a piezoelectric motor.

8. A growing prosthesis according to one of the above claims, **characterised in that** the drive (5) of the spindle element (4) has a shape-memory actuator.

9. A growing prosthesis according to one of the above claims, **characterised in that** the spindle element (4) extends in the threaded element (8) of the screw (9).

10. A growing prosthesis according to one of the above claims, **characterised in that** the threaded element (8) with thread (10) serves as a supporting means of the spindle element (4).

11. A growing prosthesis according to one of the above claims, **characterised in that** the threaded element (8) is manufactured from implant steel or ceramic or plastics material.

12. A growing prosthesis according to one of the preceding claims, **characterised in that** the drive (4) of the spindle element is capable either of being removed or installed while the implantation is ongoing.

## Revendications

1. Prothèse de croissance, comportant une partie de substitution d'articulation (1), une tige de prothèse (2), un élément d'ancrage de tige (3) et un élément avec un entraînement correspondant (5) prévu pour prolonger la prothèse de croissance, pour le prolongement étant prévu un élément de broche (4) qui est disposé dans l'élément d'ancrage de tige (3),
**caractérisée par le fait que**
l'entraînement (5) de l'élément de broche (4) est intégré dans la partie de substitution d'articulation (1), dans un alésage dans l'élément d'ancrage de tige (3) étant placé un antagoniste sous forme d'une vis (9) avec un élément de filet (8) servant d'élément de broche (4), l'élément de filet (8) étant connectée en liaison de forme à la vis (9), et servant à la fixation de l'élément de broche (4) par rapport à l'élément d'ancrage de tige (3).

2. Prothèse de croissance selon la revendication 1, **caractérisée par le fait que** la prothèse de croissance est conçue de manière modulaire.

3. Prothèse de croissance selon la revendication 1, 2 ou 3, **caractérisée par le fait que** l'élément de broche (4) et/ou l'entraînement (5) peut, lorsque l'implantation est réalisée, être placé et à nouveau retiré.

4. Prothèse de croissance selon l'une des revendications précédentes, **caractérisée par le fait que** l'élément d'ancrage de tige (3) présente un alésage destiné à recevoir l'élément de broche (4).

5. Prothèse de croissance selon la revendication 4, **caractérisée par le fait que** le diamètre de l'alésage de l'élément d'ancrage de tige (3) est choisi aussi grand que puisse être reçu un élément de broche (4) suffisamment grand en ce qui concerne l'application de force pour le prolongement de la prothèse de croissance, sans qu'il ne se produise de diminution substantielle de la résistance de l'élément d'ancrage de tige (3).

6. Prothèse de croissance selon l'une des revendications précédentes, **caractérisée par le fait que** l'entraînement (5) de l'élément de broche (4) est conçu à partir d'un moteur (11) avec un engrenage (12).

7. Prothèse de croissance selon l'une des revendications précédentes, **caractérisée par le fait que** l'entraînement (5) de l'élément de broche (4) est formé par un moteur piézo-électrique.

8. Prothèse de croissance selon l'une des revendications précédentes, **caractérisée par le fait que** l'entraînement (5) de l'élément de broche (4) présente un actionneur à mémoire de forme.

9. Prothèse de croissance selon l'une des revendications précédentes, **caractérisée par le fait que** l'élément de broche (4) s'étend dans l'élément de filet (8) de la vis (9).

10. Prothèse de croissance selon l'une des revendications précédentes, **caractérisée par le fait que** l'élément de filet (8) avec un filet (10) sert de moyen de support de l'élément de broche (4).

11. Prothèse de croissance selon l'une des revendications précédentes, **caractérisée par le fait que** l'élément de filet (8) est réalisé en acier pour implant ou en céramique ou en matière plastique.

12. Prothèse de croissance selon l'une des revendications précédentes, **caractérisée par le fait que** l'entraînement (4) de l'élément de broche peut, en cas d'implantation permanente, être alternativement retiré ou incorporé.
